# EUROPEAN PATENT APPLICATION

(11) **EP 2 090 996 A1**
(43) Date of publication of application: **19.08.2009**
(21) Application number: 08002895.4
(22) Date of filing: 16.02.2008
(51) Int. Cl.: G06F 19/00

(54) **Medical device**

(71) Applicant: Roche Diagnostics GmbH, 68305 Mannheim (DE); F. Hoffmann-La Roche AG, 4070 Basel (CH)
(72) Inventor: Rasch-Menges, Jürgen, 68726 Schwetzingen (DE); Jansen, Paul, 68163 Mannheim (DE)
(74) Representative: Twelmeier Mommer & Partner

(57) **Abstract**

The invention refers to a medical device comprising a data management unit for providing patient data regarding a patient's health and/or treatment status,a display (3) for displaying patient data provided by the data management unit, means for initiating an activation process causing activation of the device (1) and/or for initiating a deactivation process causing deactivation of the device (1). According to the invention, patient data is displayed on the display (3) in response to the initiation of an activation or a deactivation process.

## Description

The invention refers to a medical device having the features specified in the preamble of claim 1. For example, such a device may be a blood glucose meter, a glucose monitoring device, a medical data management device, an infusion device or some other medical device which collects or processes medical data regarding a patient's health and/or treatment status, especially data about glucose or other analyte concentration measurements taken and/or medication administered.

Medical measuring devices provide a concentration value of an analyte in a body fluid, for example the glucose concentration of blood or interstitial fluid, and store measured concentration values together with the time and date of measuring.

In addition to providing a concentration value modern medical devices are capable of analysing a series of measurement values, for example by calculation of an average value, sophisticated statistical evaluation or displaying the developing of concentration values over time. Such an analysis may also be provided by other devices used for the treatment of diabetics, like insulin pens or infusion devices, and is especially valuable for diabetics who need to know as much as possible about the status of their glucose metabolism for optimum treatment of their disease.

Serious long-term secondary effects of diabetes mellitus (for example blindness due to retinopathy) can be prevented only by keeping the blood glucose level within narrow limits at all times by means of exactly dosed administrations of insulin. Optimal adjustment of the dosing of insulin is hardly possible without repeated checks of the development of glucose concentration over time, i.e. an analysis of a series of measurement values of the patient's glucose concentration.

However, few diabetics use such analysis functions of their devices to the full extent. For many users accessing analysis results regarding a series of prior measurements is tedious, especially as operating controls of small hand-held devices are necessarily small and therefore difficult to handle, particularly for persons whose manual dexterity is impaired by age or disease. Often, patients are only interested in the result of a current measurement, i. e. a glucose concentration of a fluid sample just taken.

Therefore, suboptimal treatment of diabetics is often detected rather late, sometimes only after series long-term secondary effects of diabetes show up. If all users of measurement devices were to use analysis functions of their devices routinely, suboptimal treatment, especially suboptimal adjustment of insulin dosing, could be detected much sooner and harm prevented.

It is therefore an object of the present invention to help diabetics to detect problems of their glucose metabolism, especially suboptimal adjustment of insulin dosing, sooner.

This object is achieved by a medical device having the features stated in claim 1.

The deactivation of a medical device according to the invention is caused by a deactivation process which comprises a step in which patient data regarding a patient's health and/or treatment status is displayed. Thereby, patient data, for example an analysis result based on a plurality of measurements, is displayed automatically whenever a user turns the device off. To view patient data a user does not need to enter any complex commands or navigate through a menu. Hence, patient data like analysis results can be provided without any additional effort of a user.

The data management unit of a medical device according to the invention may be an analytical unit for analysing measurement values provided by a measuring unit. Such a measuring unit, e.g. for determining an anyalyte concentration of a body fluid, may be an integral part of the device, i.e. provided in a housing together with the data management unit, or be provided as a separate device communicating wirelessly or via a plug-in connection with the medical device comprising the data management unit and the display. Another possibility is that the data management unit simply stores the time of administrations of medication, e.g. insulin, and provides as patient data information about the last administration or an analysis of several administrations.

The invention facilitates a user's access to his or her patient data, e.g. analysis results based on a plurality of measurements, for example a series of measurements taken over the last few days. Moreover, patient data are provided in an unobtrusive way, although they are displayed automatically.

If a user is for any reason not interested in patient data at the time when the device is turned off, he or she can simply ignore the display during the deactivation process and put the device immediately away, for example into a pocket.

The patient data displayed in response to the initiation of the deactivation process may, for example, be based on the frequency of measurements and comprise a warning if the frequency is below a desired value. For example the number of measurements during a predefined time interval, which might be anything from a few hours up to a number of days or even weeks, can be compared with the number of measurements which should have been taken during that time interval. Diabetics often do not measure their blood glucose levels as often as required for optimum treatment of their disease. Sometimes this is the result of simple laziness, but sometimes an established schedule for measurements might be temporarily insufficient because a patient deviates from daily routine which causes changes in his or her glucose metabolism. Such a need for more frequent measurements can be detected by analysing past measurement values. The data management unit of the device can be configured to detect such a need for more frequent measurements and calculate a desired value to compare with the frequency of measurements performed. To display a warning whenever the device is turned off, is an unobtrusive way to remind users of the necessity of more frequent measurements.

Alternatively or in addition the patient data which is always displayed in response to the initiation of a deactivation process may indicate a user's metabolic status, show a diagram of previous measurement values, an average concentration value or the deviation of the present concentration value from an established average of previous measurements for that time of day.

To avoid unnecessary power consumption, the deactivation process may also be initiated automatically when no user requests have been entered for a preset time. Such a time-out function is commonly used to protect batteries of devices like cell phones or pocket calculators from depletion when a user puts the device away without turning it off. Preferably, an analysis result based on a plurality of measurements is displayed on the display in response to such an automatic initiation of the deactivation process as well as in response to an initiation by a user via operating controls.

Preferably, the deactivation process can be interrupted via the operating controls. This enables a user to access additional information from the device, if the patient data shown during the deactivation process warrants further attention or simply prodded a user's interest in other analysis results covering other aspects. For example, interruption of the deactivation process may cause the displayed analysis result to freeze or open a navigation menu offering further information.

If the deactivation process is initiated via the operating controls, it is preferred that after initiation continued actuation of the operating controls is required during an initial stage, which preferably lasts for at least half a second, especially for at least 0.8 seconds. The operation controls may for example be provided as push buttons that have then to be pressed continuously during the initial stage to prevent abortion of the deactivation process. The requirement of continued actuation during an initial stage prevents accidental deactivation of the device. After the initial stage is completed, the deactivation process takes its course without any need for further action of the user. Preferably, the analysis result is displayed during the initial stage and a secondary stage of the deactivation process thereafter.

Preferably, a progress indicator, e. g. a progress bar, is displayed during the deactivation process to indicate progression of the deactivation progress. For example, the progress indicator may indicate progression of the initial stage of the deactivation process. After the initial stage, a progress indicator may also be used during the secondary stage of the deactivation process. The progress indicator may inform a user how much longer the operation controls have to be actuated to prevent abortion of the initial stage or how much longer the analysis result will be shown before the deactivation process terminates and the device turns off.

Preferably, the analysis result is displayed during the deactivation process for at least two seconds, preferably at least four seconds. A few seconds are enough to perceive an analysis result displayed as a simple graphic or a few digits. More complex analysis results may require longer display times.

Preferably, patient data is displayed during the whole duration of the deactivation process to maximise the time a user has to view the analysis result. However, it is also possible that the deactivation process continues for a while after the display is turned off. It is also possible to display a sequence of analysis results during the deactivation process. As the amount of information that can be presented comprehensibly on a tiny display is limited, a sequence of analysis results which are displayed one after the other is an advantageous way to provide a user unobtrusively with complex information or analysis results regarding different aspects of his or her measurement history.

Preferably, the analytical unit is configured to employ different analysis procedures to provide as patient data different analysis results from a given set of concentration values and the times and dates of measurement. In this way analysis results can be provided that cover different aspects of a users glucose metabolism or measurement history. Preferably, each time the deactivation process is initiated, an analysis result provide by a different analysis procedure is displayed. Preferably, different analysis procedures of the analytical unit are used alternately to provide the analysis result that is shown during the deactivation process. As diabetics use their measurement devices several times a day, they can thus be provided with different analysis results regarding different aspects of their glucose metabolism or measurement history

Additionally, patient data is also displayed when the device is booted, i.e in response to being turned on. Such a device function may be preset by the manufacturer and could preferably be changed by a user who would not want to view analysis results prior to a measurement. Preferably, the device function that patient data is displayed in response to the initiation of a deactivation process cannot be changed by a user.

Further details and advantages of the invention are illustrated in the following by means of an exemplary embodiment and by making reference to the appended drawings. In the figures:
- Fig. 1: shows an exemplary embodiment of a measuring device according to the present invention;
- Fig. 2: shows an example of an analysis result displayed by the device during the deactivation process;
- Fig. 3: shows another example of an analysis result displayed by the device during the deactivation process;
- Fig. 4: shows another example of an analysis result displayed by the device during the deactivation process;
- Fig. 5: shows an example of a problematic analysis result provided by the same analysis procedure as the result shown in figure 4; and
- Fig. 6: shows an example of an alarming analysis result provided by the same analysis procedure as the result shown in figure 4.

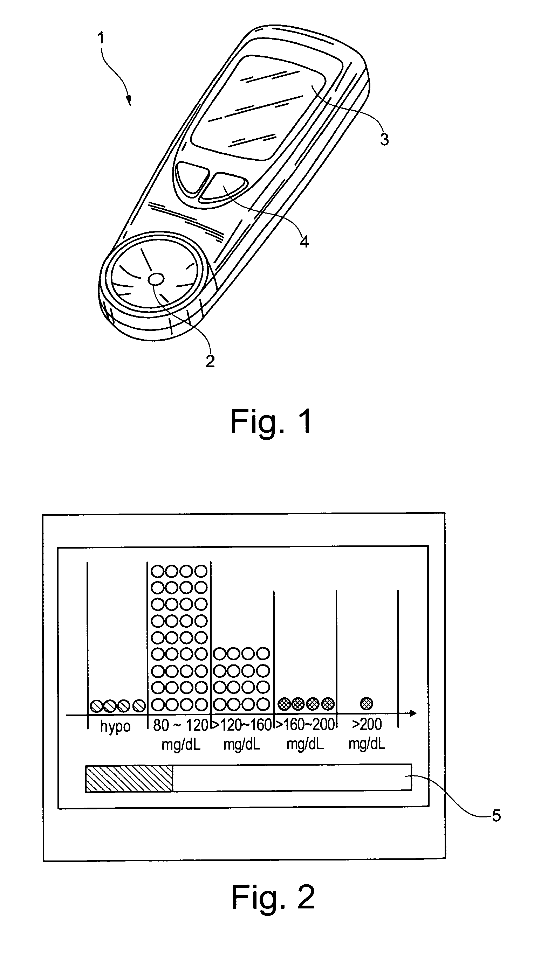

Figure 1 shows schematically a hand-held measuring device 1 for assaying a sample of a body fluid. The medical device 1 has an integrated lancing facility to puncture a user's finger to collect a sample of blood or interstitial fluid. The device 1 has an opening 2 against which a finger or other body part is pressed for puncturing. A sample collected by puncturing is assayed by a measuring unit (not shown) of the device 1 to determine an analyte concentration of the sample, e. g. its glucose concentration. Measured concentration values can be displayed on a display 3, preferably an LCD display.

Instead of having an opening 2 to be pressed against a finger for puncturing the device 1 may also be configured for use with a separate lancing device and disposable test elements, like strips, onto which a sample is applied and which are then inserted into the device for measuring. The device 1 is self-powered, preferably by batteries.

Measurement values are stored together with time and date of their measurement in a data storage of the device 1. The device 1 comprises a data management unit provided as an analytical unit (not shown) for analysing measurement values. The analytical unit may, for example, provide graphics regarding the statistical distribution of measurement values, changes of the analyte concentration over time or perform a statistical analysis of a series of measurement values. Such analysis results enable a user to detect any suboptimal adjustment of insulin dosing at a very early time and thereby helps to prevent serious long-term secondary effects of diabetes mellitus.

The device 1 has operating controls 4 by which a user can operate the device 1 and access its various functions. In the example shown the operating controls 4 are configured as two push buttons or touch keys. Via these operating elements a user can select items from a navigation menu on the display 3 and thereby view measured concentration values or analyse results.

To deactivate the device 1, i. e. turn it off, a deactivation process is initiated via the operating controls 4. The deactivation process comprises an initial stage during which the operating controls 4 have to be continuously actuated to prevent abortion of the deactivation process and a secondary stage during which no further action of a user is necessary. To deactivate the device 1 the left push button has to be pressed during the initial stage, e.g. for a minimum time of one second. Of course, it is also possible to configure the device 1 in such a way that a different button or several buttons have to be pressed during the initial stage of the deactivation process. Accidental deactivation of the device 1 is prevented by the requirement that the operating controls 4 have to be actuated continuously during the initial stage of the deactivation process.

In response to the initiation of the deactivation process an analysis result based on a plurality of measurements is displayed on the display 3. In this way a user is conveniently provided with analysis results regarding his or her metabolic status or measurement history. Preferably, an analysis result is displayed during both the initial stage of the deactivation process and during the subsequent secondary stage. An example of an analysis result displayed during the deactivation process is shown in figure 2.

Figure 2 shows a bar chart in which the length of the bars indicates the percentage of measurements which have yielded a concentration value in a concentration range assigned to each bar. Problematic ranges, i. e. concentration values which are either too low or too high for an optimum glucose metabolism may be highlighted and supplemented by a warning text message. In general, the analysis result displayed during the deactivation process may comprise graphic, symbols, numerical digits or text, for example.

The analysis result is shown during the deactivation process for four seconds, for example. Depending on the complexity of the analysis result shown, shorter or longer display times may be appropriate, for example two seconds to ten seconds.

The deactivation process can be interrupted via the operating controls 4 during the secondary stage while the analysis result is displayed. An interruption of the deactivation process may simply prevent the display 4 to turn off for a while or open a navigation menu to access other device functions, i. e. abort the deactivation process. A navigation menu displayed in response to an interrupt of the deactivation process may comprise an option to change the set up of the device, i. e. what is displayed when the device 1 is turned on. For example a user may be given the option to change the set up of the device in such a way that an analysis result like the one just such during the interrupted deactivation process is displayed whenever the device 1 is turned on. A menu option to change the set up of the device in such way may be provided only when the deactivation process has been interrupted several times during a predefined time interval, for example a week. A counter may be provided to count how often the deactivation process was interrupted.

In figure 2 the analysis result is displayed together with the progress indicator 5 in the form of a progress bar that indicates progression of the deactivation process, preferably of the initial phase. During the secondary stage another progress indicator 5 may informs a user how much time is left for interrupting the secondary stage of the deactivation process, which a user may want to do in order to access additional information regarding the analysis result shown.

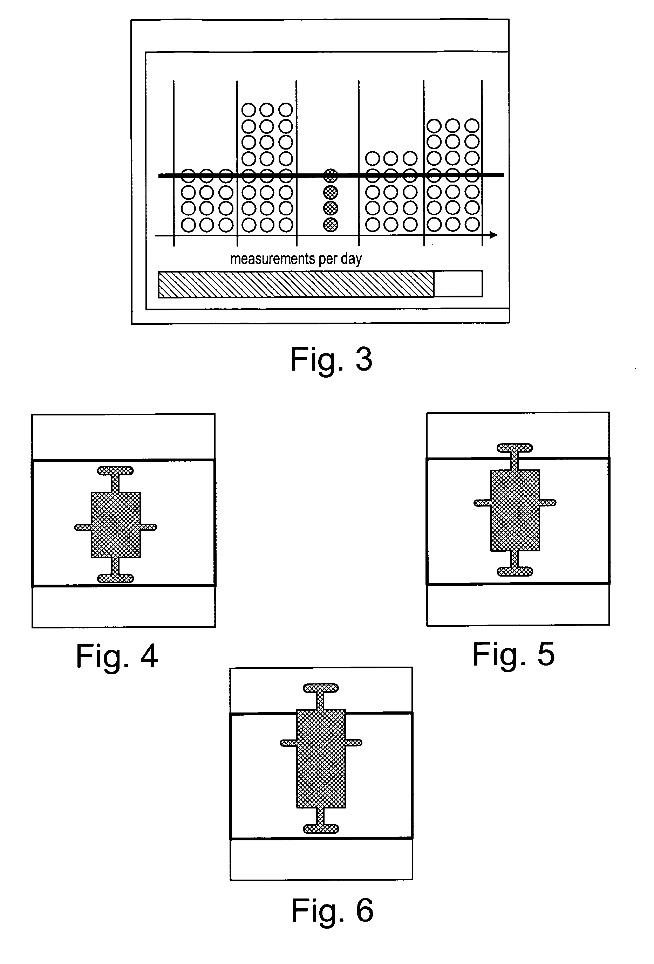

Figure 3 shows a different example of an analysis result displayed by display 3 in response to the initiation of the deactivation process. The analysis result shown in figure 3 is a bar chart in which the length of the bars corresponds to the number of measurements taken at the respective day. The right most bar refers to the current day. The other bars refer to prior days, i. e to the day before the day of the adjoining bar to their right side. The analysis result shown in figure 3 also comprises a comparison of the frequency of measurements with a desired value. The desired value is indicated by a horizontal bar in figure 3. As can be seen, this bar was superseded only on three days, whereas on two days the bar was barely reached. This indicates that on those two days additional measurements should have been taken. Especially problematic results, for example too few measurements on a given day, can be highlighted by colour, arrows or the width of the bar, for example. It is also advantageous to highlight holidays and weekends as a person's daily routine on such days is typically different from work days. The glucose metabolism may well reflect that, which warrants special attention to results regarding such days.

Figures 4, 5 and 6 show yet another example of analysis results, which were provided by a different analysis procedure. In figures 4, 5 and 6 a user's metabolic status is indicated by a symbol. In the example shown the vertical length of the symbol indicates a range of concentrations which have been measured. Concentrations which have been measured fairly often are covered by the bar, extensions of the bar reach up to the highest or lowest concentration which has been measured during the last seven days. Medically critical concentration values, especially hypo- or hyperglycaemic values are indicated by horizontal lines. In figure 4 the symbol touches none of the lines which indicates that everything is ok. In figure 5 the extension of the bar crosses the upper line which indicates that some problems exist. If the user is aware that a special situation has caused a single unusually high concentration value, the warning implied in figure 5 may be ignored. If not, the user might be well advised to change his or her lifestyle or adapted insulin dosing. In figure 6 the bar crosses the upper horizontal line which indicates that hyperglycaemic values were measured regularly. Therefore, figure 6 implies a warning that the user should consult a physician. Figures 4 to 6 may be supplemented by appropriate text messages or a background colour at reflects the metabolic status, for example the background colour may be green, yellow or red depending on the seriousness of the situation.

The device 1 may be configured such that during the deactivation process the analysis result of always the same analysis procedure is shown. It is also possible to show alternately analysis results provided by different analysis procedures. In this way, the user may be provided with the result of a different analysis procedure whenever the device 1 is turned off. For example the analysis procedures which provide diagrams according to figures 2, 3 and 4 to 6 may be used alternately. It is also possible that the device 1 chooses randomly which of its analysis procedures is used to generate the analysis result to be displayed as a response to the initiation of the deactivation process. Another possibility is that during the deactivation process a sequence of analysis results is presented, for example first a result according to figure 2 and then a result according to figure 3.

## Claims

1. Medical device comprising:
a data management unit for providing patient data regarding a patient's health and/or treatment status,
a display (3) for displaying patient data provided by the data management unit,
means (4) for initiating an activation process causing activation of the device (1) and/or for initiating a deactivation process causing deactivation of the device (1)
**characterized in that** in response to the initiation of an activation or a deactivation process patient data is displayed on the display (3).

2. Device according to claim 1, **characterized in that** the means for initiating an activation or a deactivation process comprise operating controls (4) and that the deactivation process can be interrupted via the operating controls (4) while the patient data is displayed.

3. Device according to any one of the previous claims, **characterized in that** the patient data is displayed together with a progress indicator (5) that indicates progression of the deactivation process.

4. Device according to any one of the previous claims, **characterized in that** the deactivation process comprises an initial stage, during which continued actuation of operating controls (4) is necessary to prevent abortion of the deactivation process, and a subsequent secondary stage, whereby the patient data is displayed in both stages.

5. Device according to any one of the previous claims, **characterized in that** the data management unit provides the patient data as an analysis result based on a plurality of measurement and/or medication data.

6. Device according to any one of the previous claims, **characterized in that** the patient data comprises a comparison of the frequency of measurements and a desired value.

7. Device according to any one of the previous claims, **characterized in that** the patient data indicates a patient's metabolic status.

8. Device according to any one of the previous claims, **characterized in that** the patient data comprises an analysis result based on a plurality of measurement or medication data taken over the course of at least the last 24 hours, preferably over the course of at least the previous five days.

9. Device according to any one of the previous claims, **characterized in that** the data management unit is an integral part of the device.

10. Device according to any one of the previous claims, **characterized in that** the data management unit is connected to a measuring unit for determining an analyte concentration of a sample of a body fluid.

11. Device according to claim 10, **characterized in that** the data management unit is connected to the measuring unit via a wireless or wired data communication.

12. Device according to any one of the previous claims, **characterized in that** the device is a blood glucose meter, a glucose monitoring device, a medical data management device or an infusion device.
